Europäisches Patentamt

European Patent Office

Office européen des brevets

(19)

(11) Publication number : **0 359 256 B1**

(12) **EUROPEAN PATENT SPECIFICATION**

(45) Date of publication of patent specification :
20.01.93 Bulletin 93/03

(51) Int. Cl.[5] : **A61K 31/66**

(21) Application number : **89117028.4**

(22) Date of filing : **14.09.89**

(54) **The use of inositoltrisphosphate in the treatment of tissue damage.**

(30) Priority : **15.09.88 SE 8803248**

(43) Date of publication of application :
**21.03.90 Bulletin 90/12**

(45) Publication of the grant of the patent :
**20.01.93 Bulletin 93/03**

(84) Designated Contracting States :
**AT BE CH DE ES FR GR IT LI LU NL SE**

(56) References cited :
**EP-A- 0 179 439**
**EP-A- 0 252 227**

(56) References cited :
**MED. SCI. RES., vol. 17, 16th - 30th September 1989, pages 749-750; C.J. GREEN et al.: "D-myo-inosital-1,2, 6-trisphosphate(PP56) inhibits lipid peroxidation in warm ischaemic rabbit kidneys"**
**NEUROCHEM. INT., vol. 10, no. 3, 1987, pages 361-369, Pergamon Journals Ltd, GB; G.Y. SUN et al.: "Labeling ofphosphoinositides in rat brain membranes: An assessment of changes due to post-decapitative ischemic treatment"**

(73) Proprietor : **PERSTORP AB**
**S-28480 Perstorp (SE)**

(72) Inventor : **Sirén, Matti**
**Via Poporino 9**
**CH-6926 Montagnola Lugano (CH)**

(74) Representative : **Hansen, Bernd, Dr.rer.nat. et al**
**Hoffmann, Eitle & Partner Patentanwälte**
**Arabellastrasse 4 Postfach 81 04 20**
**W-8000 München 81 (DE)**

## Description

The present invention relates to the use of at least one isomer of inositoltrisphosphate ($IP_3$) for the preparing of a medicament for preventing, alleviating or combating tissue damage in mammals including man.

Injury or destruction of tissues should be understood as damage to an aggregation of specialized cells with a particular function both internally and externally in the body, such as different organs or parts of these, vessels or skin.

Tissue damage involves a complex series of events such as dilatation of vessel walls e.g. arterioles, capillaries and venules, increased permeability of fluids including e.g. plasma proteins and increased blood flow. Increased vascular leakage often results in extravasation and oedema formation which characterize the damage of different tissues.

Often tissue damage is characterized by signs of pain, heat, redness, swelling and loss of function.

Tissue damage is not defined as a disease per se but is often a component in different diseases of both acute or chronic nature.

Damage of tissues can be induced in many ways. Inducing factors can be correlated with mechanical effects, immunological effects or chemical effects. Microorganisms such as virus, bacteria and fungus can induce tissue damage and exposure to heat, fire, radiation, cold and blows most often results in such damage. Many diseases like asthma, eczema, psoriasis, rheumatoid arthritis, diabetes and arteriosclerosis also involve different types of tissue damage.

The existing treatment of diseases connected to tissue damage are based on drugs such as non steroid antiinflammatory drugs (NSAID), steroids, antibiotics and cytostatics. In some cases also surgical therapy is used.

Existing drugs often suffer from limited effectiveness in combination with serious side effects.

Toxic effects appearing from the treatment with NSAID:s can consist of gastrointestinal side effects, allergic reactions and side effects in the central nervous system. Treatment with steroids often results in side effects such as osteoporosis and fractures, increased susceptibility to infections and peptic ulcerations.

According to the present invention it has surprisingly become possible to overcome and reduce injuries and destruction of tissues by the use of at least one isomer of inositoltrisphosphate ($IP_3$) for the preparing of a medicament for preventing, alleviating or combatting tissue damage in mammals including man, wherein the tissue damage is related to vascular leakage, burns, oedema formation, trauma or sepsis.

The invention also covers the use of inositoltrisphosphate ($IP_3$) in combination with another pharmaceutically active compound for preparing of a medicament for preventing, alleviating or combating the above mentioned tissue damage in mammals including man.

From European Patent No. 179439 and European Patent 252 227 a pharmaceutical composition comprising as a pharmaceutically active ingredient at least one isomer of inositoltrisphosphate is known. In the said patents the effect of this pharmaceutical composition is shown for different causes and areas, such as cardiovascular diseases and arthritis.

The invention relates to the use of at least one isomer of $IP_3$ for preparing a medicament for preventing, alleviating or combating for example the following conditions:

Tissue damage induced mechanically by heat and fire such as burns of first, second or third degree, oedema or trauma.

The production of $IP_3$ and the isolation of the different isomers thereof are disclosed in the US patent No 4.777.134. The $IP_3$ isomers can also be produced by synthetic methods, chemically or enzymatically, starting with e.g. inositol and a phosphorus source. Furthermore, microbiological production methods including hybrid DNA-techniques of $IP_3$ are also suitable.

The structure of $IP_3$ and the different isomers thereof are disclosed in the US patent No 4.735.936 and the US patent No 4.797.390.

It is suitable that the medicament exists in unit dosage form. Tablets, granules or capsules are suitable administration forms for such unit dosage. Furthermore, tablets and granules can easily be surface treated such as to provide an enteric coating to prevent an uncontrolled hydrolysis in the stomach and to bring about a desired absorption in the intestine. Other suitable administration forms are slow release and transdermal administration. A usual pharmaceutically acceptable additive, excipient and/or carrier can be included in the medicament. The tablets or granules can also contain a disintegrant which causes the tablets or the granules, respectively, to disintegrate easily in the intestine. In certain cases, especially in acute situations, it is preferable to use the unit dosage in the form of a solution for intravenous administration.

The medicament can also consist as such of $IP_3$ solely without any additive, excipient or carrier.

If desired, the medicament can be free of other inositol phosphates, $IP_1$, $IP_2$, $IP_4$, $IP_5$ and $IP_6$. Accordingly, the mixture of $IP_3$ isomers can have a purity of 90-100%, such as 93-100% or preferably 95-100%.

Alternatively, the medicament can consist of or comprise one or more specific $IP_3$ isomers, each present in substantially pure form. Thus, the different isomers can be isolated from each other in substantially pure form, which means that they have a purity of 80-100%, such as 82-100% or 85-100%, preferably 90-100%. Since the isomers can be produced in pure form they can be mixed in any proportion, of course.

The medicament can consist of $IP_3$, wherein said $IP_3$ is provided by at least one of $IP_6$, $IP_5$ or $IP_4$ and a degradative substance such as an enzyme suitable to form $IP_3$.

It is in most cases suitable that the $IP_3$-isomer or isomers used for the preparing of the medicament is present in salt form in order not to affect the mineral balance negatively. The salt should preferably consist of a sodium, calcium, zinc or magnesium salt or a mixture of two or more of these salts. Calcium and zinc salts or mixtures of these are especially preferred. The isomer of $IP_3$ can also partly be present as a salt of one or more physiologically acceptable compounds in the lanthanide series.

For the above mentioned reasons it is also an advantage if the medicament contains a surplus or an extra addition of at least one pharmaceutically acceptable salt of calcium, zinc or magnesium with a mineral acid or organic acid. This is especially valuable for elderly persons who are often deficient in these minerals.

For administration to human patients appropriate dosages can routinely be determined by those skilled in this art by extension of the results obtained in animals at various dosages. The preferred dosage for humans falls within the range of 0.1 to 1000 mg, especially 0.1-200 mg $IP_3$/day/kg body weight.

In animal experiments, no toxic effects were seen after administration of very high doses of $IP_3$, 160 mg/kg body weight by intraperitoneal injection to mice.

The medicament usually contains 0.01-1.5 g, such as 0.05-1.3 or preferably 0.1-1 g of $IP_3$ per unit dosage.

The amount of $IP_3$ should constitute 5 to 95 % or 15 to 80 % such as 25 to 60 % by weight of said active ingredients in the combined medicament mentioned above. In one preferred embodiment the $IP_3$ is D-myo-inositol-1.2.6-trisphosphate.

The other pharmaceutically active compound can be selected from the group of salicylates, pyrazolon derivatives, paraaminophenol derivatives, indomethacin derivatives, propionic acid derivatives, piroxicam; steroids such as glucocorticoids; gold compounds; penicillamine; antimalarials or immunosuppressive agents.

When the medicament is prepared from $IP_3$ and at least one other pharmaceutically active ingredient the dosage form could be combined or separate for separate or combined administration.

One function of the medicament is to reverse, prevent or alleviate damage to membranes of different cell types, but especially cell membranes of platelets and endothelial cells, macrophages and leucocytes such as white blood cells, lymphocytes and neutrophils. The use of the medicament results in an improved stabilization, a decreased susceptibility to deformation and an improved function of the cell types. Furthermore, parameters such as aggregability and adhesion to e.g. vessel walls are decreased.

Cellular mediators such as histamine, bradykinin, platelet activating factors and cytokines are also normalized when the medicament is used. Other results of the use of the medicament are regulation of membrane fluidity, the incorporation of components such as cholesterol and the production, incorporation and balance between different phospholipids.

Another result of the use of the medicament is the regulation of the electrolyte balance of the cell. As examples, regulation of intracellular levels of calcium, potassium, chloride and phosphate can be mentioned.

The cell surface activity and responsiveness to external stimuli via receptor activation/deactivation due to for example phosphorylation are other parameters effected by the use of the medicament.

The invention will be further explained in connection with the following examples 7-10. Examples 1-4 show the production of $IP_3$ where as examples 5 and 6 illustrate the preparation of solutions and tablets of $IP_3$. Example 7 relates to the reduction of oedema formation by treatment with $IP_3$. The preventive effect of $IP_3$ against bleeding is described in example 8 whereas example 9 teaches the beneficial use of $IP_3$ against damages caused by burns. Example 10 describes the reduction of vascular leakage when $IP_3$ is used.

Example 1

Hydrolysis of sodium phytate with baker's yeast and fractionation of a mixture of inositol phosphates.

A 1.4 kg quantity of sodium phytate (from corn, Sigma Chemical Co) was dissolved in 1 200 l sodium acetate buffer pH 4.6. 100 kg of baker's yeast from Jästbolaget, Sweden (dry substance: 28 %, nitrogen content: 2 %, phosphorus content: 0.4 %) was added with stirring and incubation was continued at 45°C. The dephosphorylation was followed by determining the inorganic phosphorus released. After 7 hours when 50 % inorganic phosphorus was liberated the hydrolysis was stopped by adding ammonia to pH 12. The suspension was centrifuged and the supernatant was collected.

800 l of the supernatant was passed through an ion-exchange column (Dowex 1, chloride form, 100 cm x 150 cm) and eluted with a linear gradient of hydrochloric acid (0-0.7 N HCL).

Example 2

Structural determination of isomers of inositoltrisphosphate.

The fraction obtained in example 1 with a phosphorus/inositol ratio of three to one was neutralized and precipitated as a calciumsalt. 100 mg of the precipitate was analyzed with H-NMR. Data show that the peak consists of myo-inositol-1.2.6-trisphosphate.

Example 3

A 0.5 gram quantity of D-chiro-inositol was dissolved in 1 ml phosphoric acid at 60°C. 20 g polyphosphoric acid was added and the mixture was heated to 150°C under vacuum for 6 hours. The mixture was diluted with water to a volume of 200 ml and passed through an ion-exchange column (Dowex 1, chloride form, 25 mm x 250 mm) and eluted with a linear gradient of hydrochloric acid (0-2.0 N HCl).

The content of the peak with the ratio of phosphorus to inositol of six to one was precipitated by addition of calciumhydroxide. The precipitate was filtered, washed and mixed with 10 ml of a cation-exchange resin to give the acid form of the inositolhexaphosphate. After neutralization with sodium hydroxide and freeze-drying the sodium salt of D-chiro-inositolhexaphosphate was obtained.

Example 4

A 0.8 gram quantity of the sodium salt of D-chiro-inositolhexaphosphate produced according to Example 3 was dissolved in 300 ml sodium acetate buffer, pH 5.2. 1.3 gram wheat phytase (EC 3.1.3.26, 0.015 U/mg from Sigma Chemical Co.) was added and the mixture was incubated at 38°C.

After the liberation of 50 % inorganic phosphorus the hydrolysis was stopped by adding ammonia to pH 12.

The mixture containing D-chiro-inositolphosphates was passed through an ion-exchange column (Dowex 1 chloride form, 25 mm x 250 mm) and eluted with a linear gradient of hydrochloric acid (0-0.7 N HCL).

The peak with the ratio of phosphorus to inositol of three to one was neutralized with 1.0 M sodium hydroxide and freeze-dried.

Stuctural determination with NMR and IR showed the product to be D-chiro-inositoltrisphosphate.

Example 5

Solution of sodium salt of D-myo-inositol-1.2.6-trisphosphate for injection.

0.5 g of the sodium salt of $IP_3$ and 0.77 g NaCl were dissolved in 98.73 ml of water for injection to form a solution suitable for injection into a person or an animal.

Example 6

Tablets of calcium salt of D-myo-inositol-1.2.6-trisphosphate.

Tablets of the calcium salt of D-myo-inositol-1.2.6-trisphosphate were produced in the following way. 50 g calcium salt of D-myo-inositol-1.2.6-trisphosphate, 132 g lactose and 6 g acacia were mixed. Purified water was then added to the mixture, whereupon the mixing was continued until a suitable consistency was obtained. The mixture was sieved and dried. Then the mixture was blended with 10 g talcum and 2 g magnesium stearate. The mixture was compressed into tablets each weighing 200 mg.

Example 7

Injection of carrageenan into the subplanar surface of the rat hind-paw induces a tissue damage that results in pronounced oedema. The degree of oedema can be reproducibly quantified by measuring paw circumference.

Two groups of five male rats were injected 2 hours and 1 hour before injection of carrageenan with 1000 mg/kg D-myo-inositol-1.2.6-trisphosphate ($IP_3$) or Krebs ringer solution (control) respectively.

Measurement of the paw diameter was made at hourly intervals.

The results show that an injection of $IP_3$ reduces the oedema with 40 % four hours after the induction of the damage as compared to control. Thus, the oedema in the $IP_3$-treated animals was strongly reduced.

Example 8

Vascular permeability and bleeding induced by histamine in the hamster cheek pouch was determined in the presence of D-myo-inositol-1.2.6-trisphosphate ($IP_3$) compared to control.

Male hamsters were fasted overnight and anaesthetised with sodium pentobarbitone. Infusion was performed through the jugular vein. Injection of histamine (2.7 mg/kg) was preceded by either saline (control) or $IP_3$ (5.0 mg/kg). The cheek pouch was observed with a microscope for a period of 80 minutes and the number of bleeding sites (petechiae) was recorded before the commencement of the infusion of histamine and at the end of the 80 min period.

The results are shown in the following table:

| Treatment | number of animals | number of petechiae | % inhibition |
|---|---|---|---|
| Saline (control) | 10 | 8.4 | - |
| $IP_3$ | 6 | 1.0 | 88 |

It can be seen that $IP_3$ is an effective compound against vascular permeability and bleeding.

Example 9

A controlled burn damage is induced in rats as a heat probe is applied to the skin of the abdomen with a constant pressure. The probe has a surface temperature of 45°C and is allowed to deliver a specific amount of energy until the temperature is lowered to 37°C. 15 minutes after the induction of the burn an injection of saline (control) or D-myo-inositol-1.2.6-trisphosphate ($IP_3$) was performed. After this first injection (10 mg/kg $IP_3$) an infusion of 0.2 mg/min $IP_3$ followed for 2 hours. 90 minutes after the damage Evans Blue is injected to the animals. This compound binds to albumin, which leaks into the damaged tissue. The amount of albumin leakage is then easily determined by measuring the intensity of colour of Evans Blue in different parts of the tissue.

After another 30 minutes the animals are killed and the tissue damage is determined. In the $IP_3$-treated animals (n=10) the burn damage was significantly reduced compared to control (n=10).

Thus $IP_3$ is an protective substance against tissue damage caused by burns.

Example 10

Local oedema formation is measured in the shaved dorsal skin of rabbits in response to mediators of increased microvascular permeability, such as histamine, bradykinin and platelet activating factor (PAF). When a vasodilator such as calcitonin gene-related peptide (CGRP) is administered together with the mediators the blood flow is increased and this system is established for testing compounds that reduces oedema formation.

Prior to injection of the different mediators Evans Blue is injected into the anaesthetized animals. The distribution of Evans Blue is a measurement of the tissue damage and oedema formation.

Directly after injection of the mediators either saline (control) or 50 mg/kg of D-myo-inositol-1.2.6-trisphosphate ($IP_3$) are performed.

The results are shown below as per cent inhibition of $IP_3$ compared to control.

| Mediator | % inhibition with $IP_3$ compared to control |
|---|---|
| Bradykinin | 64 |
| Histamine | 61 |
| PAF | 64 |

As can be seen the presence of $IP_3$ significantly reduces the oedema formation.

## Claims

1. The use of at least one isomer of inositoltrisphosphate ($IP_3$) for the preparing of a medicament for preventing, alleviating or combating tissue damage in mammals including man, wherein the tissue damage is related to vascular leakage, oedema formation, burns, trauma or sepsis.

2. The use according to claim 1, wherein said inositoltrisphosphate is in salt form.

3. The use according to claim 1 wherein said inositoltrisphosphate salt is a salt of sodium, calcium, zinc or magnesium or a mixture of two or more thereof.

4. The use according to any of claims 1 to 3, wherein the medicament is in unit dosage form comprising tablets, granules and solution.

5. The use according to claim 4, wherein the medicament is containing 0.01-1.5 g of $IP_3$ per unit dosage.

6. The use according to claim 1, wherein the isomer of inositoltrisphosphate is D-myo-inositol-1,1,6-trisphosphate.

7. The use according to claim 1, wherein the medicament is containing at least one other pharmaceutically active ingredient in addition to $IP_3$.

## Patentansprüche

1. Verwendung mindestens eines Isomeren von Inositoltrisphosphat ($IP_3$) zur Herstellung eines Medikaments zur Verhinderung, Linderung oder Bekämpfung von Gewebeschaden in Säugetieren einschließlich des Menschen, worin der Gewebeschaden verbunden ist mit Durchsickern durch die Gefäßwände, Ödembildung, Verbrennungen, Trauma oder Sepsis.

2. Verwendung gemäß Anspruch 1, worin das Inositoltrisphosphat in Salzform vorliegt.

3. Verwendung gemäß Anspruch 2, worin das Inositoltrisphosphatsalz ein Natrium-, Calcium-, Zink- oder Magnesiumsalz oder eine Mischung von zwei oder mehreren dieser Salze ist.

4. Verwendung gemäß einem der Ansprüche 1 bis 3, worin das Medikament in Dosierungseinheiten vorliegt, welche Tabletten, Granula und Lösungen umfassen.

5. Verwendung gemäß Anspruch 4, worin das Medikament 0,01-1,5 g $IP_3$ pro Dosierungseinheit enthält.

6. Verwendung gemäß Anspruch 1, worin das Inositoltrisphosphat-Isomer D-myo-inosit-1,2,6-trisphosphat ist.

7. Verwendung gemäß Anspruch 1, worin das Medikament mindestens einen anderen pharmazeutisch aktiven Bestand zusätzlich zu IP$_3$ enthält.

**Revendications**

1. Utilisation d'au moins un isomère du trisphosphate d'inositol (IP$_3$) pour la préparation d'un médicament pour empêcher, soulager ou combattre les lésions tissulaires chez les mammifères, y compris l'homme, les lésions tissulaires étant liées à une fuite vasculaire, à une formation d'oedème, à des brûlures, à un traumatisme ou à une septicémie.

2. Utilisation selon la revendication 1, dans laquelle ce trisphosphate d'inositol est sous forme de sel.

3. Utilisation selon la revendication 2, dans laquelle ce trisphosphate d'inositol est un sel de sodium, de calcium, de zinc ou de magnésium ou un mélange de deux ou plusieurs de ceux-ci.

4. Utilisation selon l'une quelconque des revendications 1 à 3, dans laquelle le médicament est sous forme de dose unitaire comprenant des comprimés, des granulés et une solution.

5. Utilisation selon la revendication 4, dans laquelle le médicament contient 0,01-1,5 g d'IP$_3$ par dose unitaire.

6. Utilisation selon la revendication 1, dans laquelle l'isomère du trisphosphate d'inositol est le 1,2,6-trisphosphate de D-myo-inositol.

7. Utilisation selon la revendication 1, dans laquelle le médicament contient au moins un autre ingrédient pharmaceutiquement actif en plus de l'IP$_3$.